# FASCICULE DE BREVET EUROPEEN

(11) **EP 0 641 194 B1**
(45) Date de publication et mention de la délivrance du brevet: **15.05.1996**
(21) Numéro de dépôt: 94909980.8
(22) Date de dépôt: 17.03.1994
(51) Int. Cl.: A61K 9/107, A61K 7/48

(54) **EMULSION HUILE-DANS-EAU CONTENANT UN PERFLUOROPOLYETHER, COMPOSITION EN COMPORTANT, PROCEDE DE PREPARATION ET UTILISATION EN COSMETIQUE ET DERMATOLOGIE**
ÖL-IN-WASSER-EMULSION, DIE EINEN PERFLUOROPOLYETHER ENTHÄLT, ZUSAMMENSETZUNG DIE DIESEN ENTHÄLT, HERSTELLUNGSMETHODE UND KOSMETOLOGISCHE UND DERMATOLOGISCHE VERWENDUNG
OIL-IN-WATER EMULSION CONTAINING A PERFLUOROPOLYETHER, COMPOSITION COMPRISING SAME, PREPARATION METHOD THEREFOR AND COSMETICOLOGICAL AND DERMATOLOGICAL USE THEREOF

(30) Priorité: 18.03.1993 FR 9303158
(43) Date de publication de la demande: 08.03.1995
(73) Titulaire: L'OREAL, F-75008 Paris (FR)
(72) Inventeur: DEPREZ, Sabine, F-91420 Morangis (FR); CANDAU, Didier, F-91570 Bièvres (FR)
(74) Mandataire: Casalonga, Axel
(86) Numéro de dépôt international: FR9400295
(87) Numéro de publication internationale: WO9421233

(56) Documents cités:
- EP-A- 0 051 526
- EP-A- 0 390 206
- EP-A- 0 391 637
- EP-A- 0 494 412
- DATABASE WPI Week 8514, Derwent Publications Ltd., London, GB; AN 85-084003 & JP,A,60 034 730 (NIPPON MECTRON KK) 22 Février 1985

## Description

La présente invention concerne des émulsions huile-dans-eau comportant un perfluoropolyéther, leur préparation, des compositions cosmétiques ou dermatologiques et leur utilisation dans le domaine cosmétique et dermatologique.

Dans le domaine des émulsions, celles à base d'un perfluoropolyéther liquide sont connues. Elles sont cependant difficiles à formuler. En effet, les perfluoropolyéthers ne sont que difficilement compatibles avec les matières premières cosmétiques usuelles : les perfluoropolyéthers sont effectivement insolubles dans l'eau et dans beaucoup de substances organiques, mises à part les substances organiques ayant une haute teneur en fluor.

On a tenté à de nombreuses reprises de pallier à ces difficultés. Ainsi, le document EP-390206 décrit une émulsion de perfluoropolyéthers liquides dans un milieu hydrophile où la phase hydrophile est essentiellement constituée d'un composé organique hydroxylé comportant au moins trois motifs hydroxyle; l'émulsion obtenue sert alors de prémélange pour l'introduction des perfluoropolyéthers. Cette méthode est difficile à mettre en oeuvre dans la mesure où elle nécessite la réalisation d'une pré-émulsion dans laquelle est introduit le perfluoropolyéther.

Par ailleurs, le document EP-494412 décrit des émulsions à base de perfluoropolyéthers et de corps gras; la stabilité y est assurée par un polyol comportant au moins trois fonctions hydroxyle, en une quantité supérieure à 10% et d'un tensio-actif soluble dans le polyol. La difficulté provient là aussi du fait que la formulation est lourde à réaliser, à cause de l'étape de préparation de la pré-émulsion. On prépare des émulsions anhydres ou quasiment anhydres et on peut ensuite y ajouter de l'eau.

Par ailleurs, des émulsions à trois phases sont décrites notamment dans les documents EP-360292, JP63-107911 et EP-422984. Le perfluoropolyéther est, dans ces émulsions, dispersé dans une émulsion eau-dans-huile ou huile-dans-eau. Leur préparation nécessite une forte agitation qui permet d'obtenir la dispersion homogène du ou des perfluoropolyéther(s). Dans ces cas, la mise en suspension des perfluoropolyéthers dans le système triphasé résulte non pas d'un phénomène thermodynamique, mais d'un phénomène mécanique; la stabilité de ces systèmes est faible dans la mesure où les gouttelettes de perfluoropolyéthers, visibles au microscope, tendent à coalescer.

La distribution non-homogène des perfluoropolyéthers huileux dans les préparations cosmétiques entraîne bien souvent une diminution des performances des perfluoropolyéthers huileux.

La demanderesse a mis en évidence un système permettant de stabiliser de façon simple les émulsions cosmétiques contenant des huiles du type perfluoropolyéther, et ceci sans faire intervenir une étape de préparation de pré-émulsion. Elles comportent en effet dès leur préparation, en une étape, une certaine quantité d'eau. Ces émulsions selon l'invention permettent d'utiliser des perfluoropolyéthers, très utiles en cosmétique pour leur effet protecteur filmogène et hydratant, et dans un pourcentage minimum efficace, sans que se posent les problèmes classiques d'homogénéité et de stabilité, problèmes qui se posent lorsque l'on a voulu, jusqu'à présent, préparer directement une émulsion huile perfluorée/eau.

La présente invention concerne une émulsion huile-dans-eau comportant au moins un perfluoropolyéther, un tensio-actif fluoré à l'exclusion des tensio-actifs fluorés cationiques, un co-émulsionnant, un alcool gras, et une phase aqueuse gélifiée.

L'émulsion stable de perfluoropolyéthers dans l'eau est réalisable selon l'invention par la stabilisation qui est réalisée par la présence des quatre constituants, agissant en synergie.

La demanderesse a ainsi mis en évidence que des formulations préparées de façon comparative en n'utilisant, outre le perfluoropolyéther et l'eau, que l'un, deux ou trois des composants parmi le gélifiant, le tensio-actif fluoré, le co-émulsionnant et l'alcool, ne menaient pas à une émulsion fine et stable.

Le système émulsionnant, selon l'invention, permet d'incorporer de grandes quantités de perfluoropolyéthers, par rapport aux quantités usuellement incorporées dans les compositions plus ou moins stables connues, tout en restant stables, homogènes et ces émulsions sont obtenues directement.

Ces émulsions comportent par ailleurs de bonnes propriétés sensorielles liées à la présence de perfluoropolyéthers : elles sont en effet confortables, très faciles à appliquer et conduisent à la formation d'un film de très faible épaisseur, très doux, uniforme et ayant de bonnes propriétés de protection et de tenue.

Selon l'invention, de préférence, le tensio-actif fluoré est utilisé en des quantités allant de 0,5 à 10% en poids par rapport au poids total de l'émulsion, et de préférence de 1 à 3% en poids.

Selon l'invention, le co-émulsionnant est de préférence hydrocarboné; il est utilisé dans des pourcentages allant de 0,1 à 5% en poids par rapport au poids total de la composition, et de préférence de 0,1 à 0,3%.

L'alcool gras est utilisé, selon l'invention, dans une quantité allant de 0,5 à 10% en poids, et de préférence de 1 à 3% en poids par rapport au poids total de la composition.

La phase aqueuse gélifiée est utilisée, selon l'invention, dans une quantité pouvant aller de 10 à 90% en poids par rapport au poids total de l'émulsion.

Les perfluoropolyéthers ou leurs mélanges sont quant à eux utilisés dans des quantités allant de 0,5 à 50% en poids par rapport au poids total de la composition, et de préférence de 5 à 20% en poids.

Selon l'invention, les perfluoropolyéthers liquides mis en oeuvre dans les émulsions, peuvent être du type fonctionnalisé ou du type non fonctionnalisé.

Parmi les perfluoropolyéthers non fonctionnalisés, on peut citer notamment les composés de formule (I) : 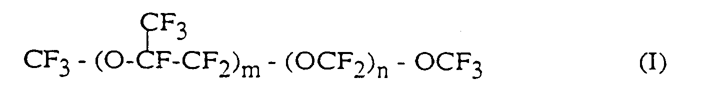
dans laquelle m/n = 5 à 40, et m et n sont choisis de telle sorte que le poids moléculaire moyen soit supérieur à 500 et de préférence compris entre 1.000 et 10.000.

Parmi ceux-ci, on peut citer ceux qui sont vendus sous les dénominations de "FOMBLIN HC", de "FOMBLIN Y", de "FOMBLIN HCR" (poids moléculaire : 6250), de "FOMBLIN HC-04" (poids moléculaire : 1500), de "FOMBLIN HC-25" (poids moléculaire : 3200), et de "GALDEN" par la Société MONTEFLUOS. On peut citer aussi les composés de formule (II) suivante :

CH₃-(O-CF₂-CF₂)ₚ-(OCF₂)_{q}-OCF₃ (II)

dans laquelle p/q est de 0,5 à 1,5, le poids moléculaire moyen étant supérieur à 500 et de préférence compris entre 1.000 et 10.000.

Parmi ceux-ci, on peut citer le composé vendu sous la dénomination de "FOMBLIN Z" par la Société MONTEFLUOS.

On peut citer encore les composés de formule (III) suivante : 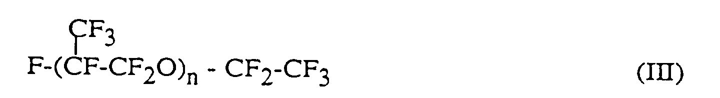
dans laquelle n est un nombre entier de 4 à 500.

Parmi ceux-ci, on peut citer le composé vendu sous la dénomination de "KRYTOX" par la Société DU PONT DE NEMOURS.

On peut citer enfin les composés ayant la formule (IV) suivante : 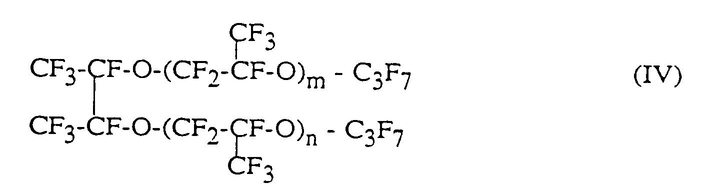
dans laquelle n et m sont des nombres entiers de 0 à 3.

Parmi ceux-ci, on peut citer les composés vendus sous la dénomination de "HOSTINERT" par la Société HOECHST.

Parmi les perfluoropolyéthers fonctionnalisés, on peut citer les composés ayant la formule suivante :

RCF₂-(O-CF₂CF₂)ₚ-(OCF₂)_{q}-OCF₂R (V)

dans laquelle :
p/q est de 0,5 à 1,5, et
R représente un reste -COOCH₃, -CH₂OH,
-CH₂O-CH₂-CHOHCH₂OH ou -CH₂-(OCH₂-CH₂)ₜ -OH où t est 1 ou 2, le poids moléculaire moyen étant supérieur à 500 et de préférence compris entre 1.000 et 10.000.

Parmi ceux-ci, on peut citer les composés vendus sous les dénomination de "FOMBLIN Z-DOL" (R = -CH₂OH), "FOMBLIN Z TETRAOL" (R = CH₂O-CH₂-CHOHCH₂OH) et de "FOMBLIN Z-DOL-TX" [(R=CH₂(OCH₂CH₂)ₜ OH, t étant 1 ou 2)] par la Société MONTEFLUOS.

A titre de tensio-actifs fluorés à l'exclusion des tensio-actifs fluorés cationiques, on peut utiliser, selon l'invention :
- les tensio-actifs fluoroalkylpolyglycérolés de formule (VI) :

   R_{f}-(CH₂)ₘ-S-Gₙ-H (VI)

   dans laquelle :
   R_{f} désigne un radical alkyle perfluoré, linéaire ou ramifié, en C₆ à C₂₀ ou un mélange de radicaux alkyle perfluorés, linéaires ou ramifiés, en C₄ à C₂₀;
   m représente 0, 1 ou 2;
   n représente une valeur statistique ou entière comprise entre 1 et 10;
   G représente un motif choisi parmi : 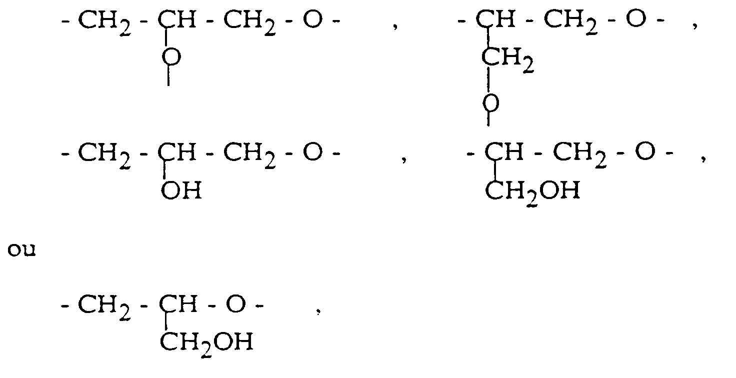 chacun des atomes d'oxygène pouvant être relié à un atome d'hydrogène ou à un autre motif G; dans la formule (VI), de préférence, R_{f} désigne un radical alkyle perfluoré, linéaire ou ramifié en C₆ à C₁₄ et m est 2; ou
- les perfluoroalkyles oxyéthylénés ou oxypropylénés de formule (VII) : 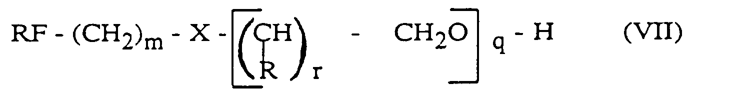 où
   . RF est un groupement perfluoroalkyle CₙF₂ₙ₊₁ où n est compris entre 3 et 20;
   . m = 0, 1 ou 2, de préférence 2;
   . q = 1 à 10, de préférence 2;
   . R est un groupement méthyle ou hydrogène;
   . r = 1 lorsque R = CH₃ et r = 1 ou 2 lorsque R = H;
   . X est un atome d'oxygène ou de soufre.

On peut utiliser en particulier les composés vendus sous la dénomination "ZONYL FSN" et "ZONYL FSN 100" par la Société DUPONT.

Les composés de formule (VI) sont décrits dans la demande de brevet français n° 92-09404 déposée le 29 juillet 1992.

Ces composés de formule (VI) peuvent être préparés en mettant en oeuvre la réaction d'un mercaptan fluoré de formule (VIII) :

R_{f}-(CH₂)ₘ-S-H (VIII)

dans laquelle R_{f} et m ont les mêmes significations que dans la formule (VI) en présence d'une quantité active de catalyseur basique,
a) par condensation avec n moles
   - de glycidol, ou
   - d'un composé à fonction époxyde susceptible, après réaction, de régénérer une fonction alcool,
      éventuellement suivie d'une neutralisation ; ou
b) sur l'éther de glycidyle et d'isopropylidèneglycéryle, quand n valeur entière, est égale à 2 ou à un multiple de 2,
   éventuellement suivie d'une hydrolyse ; ou
c) sur l'éther de glycidyle et de diisopropylidènetriglycéryle, quand n est une valeur entière égale à 4,
   éventuellement suivie d'une hydrolyse.

Certains tensio-actifs fluorés cationiques ne conviennent pas. En particulier, certains tensio-actifs ammonium quaternaire comme le composé connu sous le nom de "LODYNE S106B" de CIBA, ne conviennent pas.

Parmi les co-émulsionnants hydrocarbonés utilisables dans les émulsions selon l'invention, on peut citer :
a) les copolymères oxyde d'éthylène-oxyde de propylène, et en particulier ceux vendus sous le nom de "SYMPERONIC" par la Société ICI, qui ont pour formule générale : 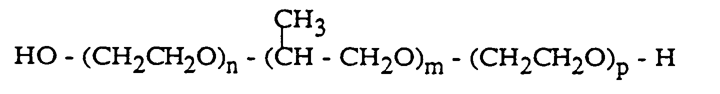 où m, n et p ont des valeurs de 2 à 100,
   et plus particulièrement le SYMPERONIC PE/F68 où n = 75, m = 30 et p = 75 ; et
b) les éthers en C₄-C₂₀ polyoxyéthylénés et/ou polyoxypropylénés, par exemple le PPG26 Butheth 26 de la Société WITCO, ou le Steareth 100 (Brij 700) de la Société ICI.

Comme alcools gras utilisables selon l'invention, on peut citer tous les alcools à chaîne hydrocarbonée comprise entre C₁₂ et C₂₂ et en particulier l'alcool cétylique, le laurylglycol commercialisé notamment par la Société CHIMEX sous la dénomination "MEXANYL GU", ou l'octyldodécanol vendu sous la dénomination d"'ISOFOL 20F" par la Société CONDEA.

En ce qui concerne la phase aqueuse gélifiée, la teneur en gélifiant dépend de la nature du gélifiant et de la matière première utilisée. C'est pourquoi la quantité en gélifiant peut varier de 0,1 à 60% en poids par rapport au poids total de la composition. On peut utiliser en effet par exemple 0,5% d'une poudre et jusqu'à environ 60% d'un gel aqueux dont le taux en matière active est usuellement beaucoup plus faible.

Parmi les gélifiants aqueux selon l'invention, on peut citer :
- les celluloses modifiées telles que l'hydroxyéthylcellulose, la méthylcellulose, l'hydroxypropylcellulose et la carboxyméthylcellulose. Parmi celles-ci, on peut citer notamment les gommes vendues sous la dénomination "CELLOSIZE QP 4400H" par la Société AMERCHOL;
- la gomme de caroube, la gomme de guar, la gomme de guar quaternisée vendue sous la dénomination "JAGUAR C-13-S" par la Société MEYHALL, la gomme d'hydroxypropylguar, la gomme de xanthane;
- les acides polyacryliques réticulés tels que les CARBOPOLS de la Société GOODRICH;
- les polymères poly(méth)acrylates de glycéryl, vendus sous les dénominations "HISPAGEL" ou "LUBRAGEL" par les Sociétés HISPANO QUIMICA ou GUARDIAN;
- la polyvinylpyrrolidone, l'alcool polyvinylique;
- les polymères réticulés d'acrylamide et d'acrylate d'ammonium, vendus sous les dénominations "PAS 5161" ou "BOZEPOL C" par la Société HOECHST, les polymères réticulés d'acrylamide et d'acide 2-acrylamido 2-méthylpropane sulfonique partiellement ou totalement neutralisé, vendus sous la référence "SEPIGEL 305" par la Société SEPPIC, les polymères réticulés d'acrylamide et de chlorure de methacryloyloxyéthyltriméthylammonium, vendus sous la référence "SALCARE SC92" par la Société ALLIED COLLOIDS; ou encore
- les homopolymères réticulés de chlorure de méthacryloyloxyéthyltriméthylammonium, vendus sous la référence "SALCARE SC95" par la Société ALLIED COLLOIDS.

Selon l'invention, la phase grasse de l'émulsion comporte les perfluoropolyéthers cités plus haut ainsi que le ou les alcool(s) gras, mais elle peut en outre comporter, sous forme émulsionnée dans la phase aqueuse, d'autres huiles usuellement employées en cosmétologie. Ainsi, la phase grasse peut comporter en outre des huiles et cires hydrocarbonées comme le squalane, les paraffines liquides, la vaseline, l'huile de parléam, la lanoline; des esters d'acides gras comme le palmitate de 2-éthylhexyle, le myristate d'isopropyle ou l'hexanoate de 2-cétyléthyle; des triglycérides comme l'huile de jojoba, l'huile de sésame, l'huile d'avocat, l'huile d'abricot ou les triglycérides synthétiques d'acides gras en C₈-C₁₈; des cires minérales comme l'ozokérite, les paraffines, ou la cérésine; des cires végétales ou animales comme la cire de Carnauba ou la cire d'abeilles; des cires synthétiques comme les cires de polyéthylène; des silicones comme les diméthylpolysiloxanes cycliques, les diméthylpolysiloxanes de faible et/ou de haute viscosité, les gommes de silicone, des organopolysiloxanes comme les phénylméthylpolysiloxanes, les alkylméthylpolysiloxanes, les alcoxyméthylpolysiloxanes, des silicones comportant des groupements fonctionnels comme les fonctions alcool ou amine ou thiol, les silicones fluorées.

Parmi les silicones fluorées, on peut citer celles ayant la formule suivante : 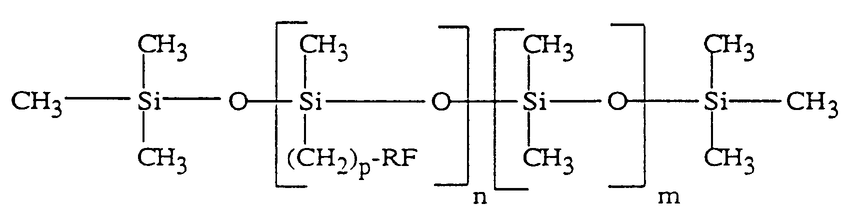
dans laquelle :
n est un nombre entier de 1 à 300,
m est un nombre entier de 0 à 150,
p est un nombre entier de 0 à 5, et
RF est un radical perfluoroalkyle ayant de 1 à 8 atomes de carbone.

Parmi les silicones fluorées, on peut citer celles vendues par la Société SHIN-ETSU sous les dénominations de "FL-100", "X 22819", "X 22820", "X 22821" et "X 22822", ainsi que celles vendues par la Société DOW CORNING sous la dénomination de "FS 1265" et celles vendues par la Société GENERAL ELECTRIC sous la dénomination de "FF 150".

On peut également utiliser des huiles perfluorées comme par exemple les perfluoroalcanes, les perfluorocycloalcanes, les perfluoro (alkylcycloalcanes), les hydrocarbures perfluorés aromatiques ou les hydrocarbures perfluorés contenant au moins un hétéro-atome, comme les amines tertiaires, les composés hétérocycliques saturés.

La phase grasse des émulsions selon l'invention peut encore comprendre des filtres, des vitamines, des hormones, des actifs cosmétiques, des antioxydants, des conservateurs, des colorants, des parfums et des filtres solaires liposolubles.

Cette phase grasse constitue la phase émulsionnée dans la phase aqueuse.

La phase aqueuse, gélifiée, pourra contenir outre l'eau, le tensioactif fluoré, le coémulsionnant hydrocarboné, également d'autres constituants habituellement utilisés dans le domaine cosmétique.

A titre de constituants supplémentaires que l'on peut incorporer à la phase aqueuse, on peut citer des polyols comme le propylèneglycol, le butylène 1,3-glycol, le glycérol et le polyglycérol, le sorbitol, le glucose ou encore la saccharose; des agents actifs tels que l'acide hyaluronique, le hyaluronate de sodium, le pyroglutamate de sodium, le gluconate de magnésium, des oligo-éléments et des dérivés biologiques; des acides aminés, des colorants ou des filtres solaires hydrosolubles.

On peut encore citer à titre de constituants supplémentaires :
- les poudres végétales telles que l'amidon de maïs, de froment ou de riz,
- les poudres minérales telles que le talc, le kaolin, le mica, la silice, les silicates, l'alumine, les zéolites, l'hydroxyapatite, la séricite, le dioxyde de titane, les micatitanes, l'oxyde de zinc, le sulfate de baryum, les oxydes de fer, le violet de manganèse, l'oxyde de chrome, le bleu d'outremer et l'oxychlorure de bismuth ou encore le nitrure de bore,
- les poudres métalliques telles que la poudre d'aluminium,
- les poudres organiques telles que les poudres de nylon, les poudres de polyamide, les poudres de polyester, les poudres de cellulose, les poudres de polyéthylène, les poudres de polypropylène, les poudres de polystyrène et les poudres de polytétrafluoroéthylène,
- les pigments organométalliques associant le zirconium, le baryum ou l'aluminium à des colorants organiques.

Lorsqu'on utilise des produits pulvérulents tels que ceux cités ci-dessus, ceux-ci peuvent être éventuellement enrobés par des sels métalliques d'acides gras, des acides aminés, de la lécithine, du collagène, du polyéthylène, des composés siliconés, des composés fluorés, des composés fluorosiliconés.

Ainsi, la présente invention concerne un procédé de préparation des émulsions huile-dans-eau selon l'invention, caractérisé en ce que :
- on disperse le tensio-actif fluoré et le co-émulsionnant hydrocarboné dans la phase aqueuse,
- on ajoute le gélifiant aqueux sous agitation vigoureuse,
- on chauffe le (ou les) perfluoropolyéther(s), l'alcool gras et les éventuels adjuvants de la phase grasse, et
- on incorpore la phase grasse dans la phase aqueuse sous agitation vigoureuse.

Pour préparer les émulsions selon l'invention, on disperse le tensio-actif fluoré et le co-émulsionnant hydrocarboné dans l'eau à 80°C, par exemple, au moyen d'un agitateur à haut degré de cisaillement de type "polytron".

On ajoute alors le gélifiant aqueux sous agitation vigoureuse, et on chauffe tous les ingrédients de la phase grasse, y compris le (ou les) perfluoropolyéther(s) à 80°C. On incorpore la phase grasse dans la phase aqueuse sous agitation vigoureuse comme une émulsion huile-dans-eau classique.

L'émulsion est ensuite refroidie progressivement à la température ambiante tout en maintenant l'agitation.

Les émulsions ainsi obtenues sont stables pendant plusieurs mois dans une large gamme de températures comprises entre + 4°C et 45°C et résistent à l'épreuve de centrifugation de 4.000 tours/minute pendant 1 heure.

Les émulsions préparées selon l'invention peuvent se présenter, à température ambiante, sous différents aspects physiques liés notamment à la nature des constituants présents dans chacune des phases et à la proportion respective de ces dernières. Il est ainsi possible d'obtenir des résultats de viscosité très différents allant du très fluide au moins fluide, en agissant sur le pourcentage de la phase aqueuse par rapport à la phase grasse et/ou encore en choisissant des constituants viscosifiants ou structurants dans chacune des phases.

Compte tenu des bonnes propriétés sensorielles des émulsions réalisées, elles trouvent de multiples applications dans le domaine cosmétique et permettent d'obtenir des produits blancs et des produits colorés.

Ainsi, la présente invention concerne l'utilisation des émulsions de l'invention pour la préparation de compositions cosmétiques ou dermatologiques.

Elle concerne également les compositions cosmétiques ou dermatologiques comportant au moins une émulsion de l'invention.

On peut ainsi présenter les émulsions selon l'invention dans des compositions sous forme de lait, de crème blanche, de crème de soin ou de crème anti-solaire, de crème teintée, de fond de teint ou de mascara.

Des procédés de traitement cosmétique de la peau et de ses phanères peuvent être mis en oeuvre par l'application des émulsions et/ou compositions de l'invention.

D'autres avantages et caractéristiques de l'invention apparaîtront à la lecture des exemples ci-après.

### EXEMPLE DE PREPARATION DU TENSIO-ACTIF FLUORE

### EXEMPLE A

Tensio-actif fluoré de formule :

C₈F₁₇-(CH₂)₂-S-G₃-H

G représentant un motif choisi parmi : 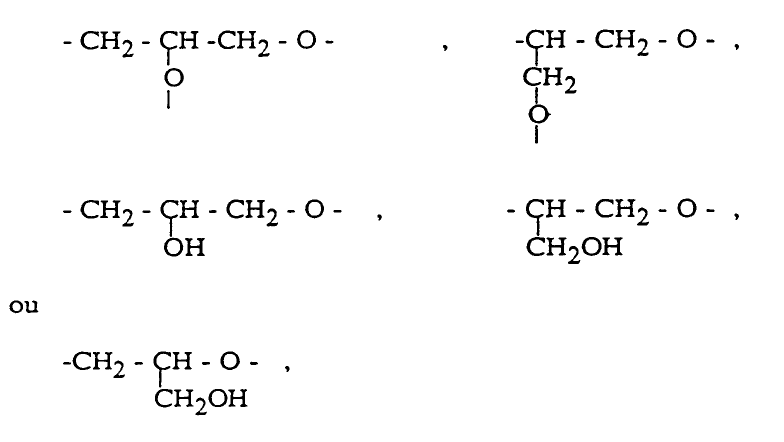
chacun des atomes d'oxygène pouvant être relié à un atome d'hydrogène ou à un autre motif G.

Ce composé est préparé selon le mode opératoire suivant :
Dans un réacteur sous atmosphère inerte sont introduits 108 g (0,225 mole) de 2-F-octyléthanethiol. Sous agitation et à la température de 25°C, on ajoute 1,26 g de tertiobutylate de potassium (11,25 meq). La température est amenée entre 50 et 60°C et le montage est placé sous un vide de 4000 Pa afin d'éliminer le tertiobutanol. La montée en température est poursuivie. Lorsque l'on atteint 80°C, on additionne en 30 minutes 16,65 g de glycidol (0,225 mole) en maintenant une température voisine de 80°C. Le milieu réactionnel devient pâteux. Afin de fluidifier le mélange, 59 g de xylène sont ajoutés en 15 minutes. Le chauffage est repris jusqu'à ce que la température atteigne 130°C.

A cette température, on ajoute en 75 minutes 33,3 g de glycidol (0,45 mole) goutte à goutte. A la fin de l'addition, la température est maintenue à 130°C pendant 15 minutes. La montée en température est reprise jusqu'à 155°C et le xylène est éliminé par distillation à pression atmosphérique.

Après 15 minutes à 155°C, le milieu est neutralisé par 11,5 ml de HCl 1N. Quelques mousses se forment puis le mélange devient translucide, la température a chuté à 135°C. A cette température, le produit est laissé environ 15 minutes sous 4000 Pa puis se solidifie à la température ambiante.

On obtient 157 g de produit.

Point de fusion : 64°C

| **ANALYSE ELEMENTAIRE** | | | | |
|---|---|---|---|---|
| | % C | % H | % S | % F |
| Calculé | 32,48 | 3,27 | 4,56 | 46,01 |
| Trouvé | 32,47 | 3,27 | 4,28 | 46,16 |

### EXEMPLES D'EMULSION HUILE-DANS-EAU

### EXEMPLE 1

| **Crème de soin** | |
|---|---|
| - Polyacrylate de glycéryle, vendu sous la dénomination d'"HISPAGEL-100" par la Société HISPANO QUIMICA | 56,14 g |
| - Polyfluoroalcool oxyéthyléné, vendu sous la dénomination de "ZONYL FSN 100" par la Société DU PONT DE NEMOURS | 2,4 g |
| - Condensat oxyde d'éthylène, oxyde de propylène, vendu sous la dénomination de "SYMPERONIC PE/F68" par la Société ICI | 0,26 g |
| - Alcool cétylique vendu sous la dénomination de "SIPOL C16" par la Société HENKEL | 3 g |
| - Perfluoropolyéthers vendus sous la dénomination de "FOMBLIN-HCR" par la Société MONTEFLUOS | 15,4 g |
| - Huile d'abricot | 5 g |
| - Huile de sésame | 1,5 g |
| - Triglycéride caprylique/caprique, vendu sous la dénomination de "MIGLYOL 812" par la Société HULS | 1,5 g |
| - Eau et conservateurs | 14,8 g |

### EXEMPLE 2

| **Crème de soin** | |
|---|---|
| - Polyacrylate de glycéryle, vendu sous la dénomination d'"HISPAGEL-100" par la Société HISPANO QUIMICA | 56,14 g |
| - Tensio-actif fluoré de l'exemple A | 2,4 g |
| - Ether polyoxypropyléné polyoxyéthyléné de butanol, vendu sous la dénomination de "WITCONOL APEB" par la Société WITCO | 0,26 g |
| - Alcool cétylique vendu sous la dénomination de "SIPOL C16" par la Société HENKEL | 3 g |
| - Perfluoropolyéther vendu sous la dénomination de " FOMBLIN-HCR" par la Société MONTEFLUOS | 15,4 g |
| - Eau et conservateurs | 22,8 g |

### EXEMPLE 3

| **Crème de soin** | |
|---|---|
| - Polyacrylate de glycéryle, vendu sous la dénomination d'"HISPAGEL-100" par la Société HISPANO QUIMICA | 56,14 g |
| - Tensio-actif fluoré de l'exemple A | 2,4 g |
| - Condensat oxyde d'éthylène, oxyde de propylène, vendu sous la dénomination de "SYMPERONIC PE/F68" par la Société ICI | 0,26 g |
| - Alcool cétylique vendu sous la dénomination de "SIPOL C16" par la Société HENKEL | 3 g |
| - Homopolymère d'hexafluoropropylène époxyde, vendu sous la dénomination de "KRYTOX 143 AC" par la Société DU PONT DE NEMOURS | 15,4 g |
| - Eau et conservateurs | 22,8 g |

### EXEMPLE 4

| **Crème de soin** | |
|---|---|
| - Polymère carboxyvinylique, vendu sous la dénomination de "SYNTHALEN K" par la Société SIGMA | 0,5 g |
| - Tensio-actif fluoré de l'exemple A | 2,4 g |
| - Condensat oxyde d'éthylène, oxyde de propylène, vendu sous la dénomination de "SYMPERONIC PE/F68" par la Société ICI | 0,26 g |
| - Alcool cétylique vendu sous la dénomination de "SIPOL C16" par la Société HENKEL | 3 g |
| - Perfluoropolyéther vendu sous la dénomination de "FOMBLIN-HC" par la Société MONTEFLUOS | 15,4 g |
| - Triéthanolamine | 0,5 g |
| - Glycérine | 5 g |
| - Eau et conservateurs | 72,94 g |

### EXEMPLE 5

| **Crème de soin** | |
|---|---|
| - Polyméthacrylate de glycéryle, vendu sous la dénomination de "LUBRAGEL" par la Société GUARDIAN | 56,14 g |
| - Tensio-actif fluoré de l'exemple A | 2,4 g |
| - Condensat oxyde d'éthylène, oxyde de propylène, vendu sous la dénomination de "STEARETH 100" par la Société ICI | 0,26 g |
| - Dodécanediol vendu sous la dénomination de "MEXANYL GU" par la Société CHIMEX | 3 g |
| - Perfluoropolyéther vendu sous la dénomination de "FOMBLIN-HCR" par la Société MONTEFLUOS | 15,4 g |
| - Eau et conservateurs | 22,8 g |

### EXEMPLE 6

| **Fond de teint** | |
|---|---|
| - Polyacrylate de glycéryle, vendu sous la dénomination d'"HISPAGEL-100" par la Société HISPANO QUIMICA | 53,14 g |
| - Tensio-actif fluoré de l'exemple A | 2,4 g |
| - Condensat oxyde d'éthylène, oxyde de propylène, vendu sous la dénomination de "SYMPERONIC PE/F68" par la Société ICI | 0,26 g |
| - Alcool cétylique vendu sous la dénomination de "SIPOL C16" par la Société HENKEL | 3 g |
| - Perfluoropolyéther vendu sous la dénomination de "GALDEN D03" par la Société MONTEFLUOS | 15,4 g |
| - Huile d'abricot | 5 g |
| - Huile de sésame | 1,5 g |
| - Triglycéride caprylique/caprique, vendu sous la dénomination de "MIGLYOL 812" par la Société HULS | 1,5 g |
| - Oxyde de titane | 4,8 g |
| - Pigment noir vendu sous la dénomination de "SICOMET Noir" par la Société BASF | 0,22 g |
| - Pigment jaune vendu sous la dénomination de "SICOMET Jaune-10" par la Société BASF | 4,43 g |
| - Pigment rouge vendu sous la dénomination de "SICOMET Rouge" par la Société BASF | 0,55 g |
| - Eau et conservateurs | 7,8 g |

### EXEMPLE 7

| **Lait** **Phase A** | |
|---|---|
| - Polyméthacrylate de glycéryle, vendu sous la dénomination de "LUBRAGEL" par la Société GUARDIAN | 28,07 g |
| - Tensio-actif fluoré de l'exemple A | 1,2 g |
| - Condensat oxyde d'éthylène, oxyde de propylène, vendu sous la dénomination de "SYMPERONIC PE/F68" par la Société ICI | 0,13 g |
| - Eau | 8,4 g |

| **Phase B** | |
|---|---|
| - Alcool cétylique vendu sous la dénomination de "SIPOL C16" par la Société HENKEL | 0,5 g |
| - Perfluoropolyéther vendu sous la dénomination de "FOMBLIN-HC" par la Société MONTEFLUOS | 7,7 g |
| - Huile d'abricot | 2,5 g |
| - Huile de sésame | 0,75 g |
| - Triglycéride caprylique/caprique, vendu sous la dénomination de "MIGLYOL 812" par la Société HULS | 0,75 g |

| **Phase C** | |
|---|---|
| - Eau | 47,5 g |
| - Dodécanediol polyglycérolé, vendu sous la dénomination de "CHIMEXANE NF" par la Société CHIMEX | 2.5 g |
| Pour préparer le lait, on verse la phase **B** dans la phase **A**, puis on y ajoute la phase **C**. | |

### EXEMPLE 8

| **Crème de soin** | |
|---|---|
| - Tensio-actif fluoré de l'exemple A | 10 g |
| - Condensat oxyde d'éthylène, oxyde de propylène, vendu sous la dénomination de "SYMPERONIC PE/F68" par la Société ICI | 1 g |
| - Méthylparabène | 0,2 g |
| - Eau | 17,6 g |
| - Polyacrylate de glycéryle, vendu sous la dénomination d'"HISPAGEL 100" par la Société HISPANO QUIMICA | 40 g |
| - Alcool cétylique | 1 g |
| - Perfluoropolyéther vendu sous la dénomination de "FOMBLIN HCR" par la Société MONTEFLUOS | 30 g |
| - Propylparabène | 0,2 g |
| On obtient une crème beige en fine émulsion. | |

### EXEMPLE 9

| **Crème de soin** | |
|---|---|
| - Tensio-actif fluoré de l'exemple A | 2,5 g |
| - Condensat oxyde d'éthylène, oxyde de propylène, vendu sous la dénomination de "SYNPERONIC PE/F68" par la Société ICI | 0,26 g |
| - Méthylparabène | 0,2 g |
| - Eau | 20 g |
| - Polyacrylate de glycéryle, vendu sous la dénomination d'"HISPAGEL 100" par la Société HISPANO QUIMICA | 55 g |
| - Alcool cétylique | 1 g |
| - Perfluoropolyéther vendu sous la dénomination de "FOMBLIN HCR" par la Société MONTEFLUOS | 1 g |
| - Huile d'abricot | 10 g |
| - Huile de sésame | 4,92 g |
| - Triglycérides d'acides caprique/caprylique, vendus sous la dénomination de "MIGLYOL 812" par la Société DYNAMIT NOBEL | 4,92 g |
| - Propylparabène | 0,2 g |
| On obtient une crème blanche en émulsion très fine. | |

### EXEMPLE 10

| **Crème de soin** | |
|---|---|
| - Tensio-actif fluoré de l'exemple A | 10 g |
| - Condensat oxyde d'éthylène, oxyde de propylène, vendu sous la dénomination de "SYNPERONIC PE/F68" par la Société ICI | 1 g |
| - Conservateurs | 0,4 g |
| - Eau | 16,6 g |
| - Polyacrylate de glycéryle, vendu sous la dénomination d'"HISPAGEL 100" par la Société HISPANO QUIMICA | 56 g |
| - Alcool cétylique | 1 g |
| - Perfluoropolyéther vendu sous la dénomination de "FOMBLIN HCR" par la Société MONTEFLUOS | 15 g |
| On obtient une crème beige en fine émulsion. | |

## Revendications

1. Emulsion huile-dans-eau, caractérisée en ce qu'elle comporte au moins :
- un perfluoropolyéther,
- un tensio-actif fluoré, à l'exclusion de tout tensio-actif fluoré cationique,
- un co-émulsionnant,
- un alcool gras,
- une phase aqueuse gélifiée.

2. Emulsion selon la revendication 1, caractérisée en ce qu'elle comporte de 0,5 à 10% en poids d'au moins un tensio-actif fluoré par rapport au poids total de la composition, et de préférence de 1 à 3% en poids.

3. Emulsion selon la revendication 1 ou 2, caractérisée en ce qu'elle comporte de 0,1 à 5% en poids par rapport au poids total de la composition d'au moins un co-émulsionnant hydrocarboné, et de préférence de 0,1 à 0,3% en poids.

4. Emulsion selon l'une des revendications 1 à 3, caractérisée en ce qu'elle comporte de 0,5 à 10% en poids par rapport au poids total de la composition d'au moins un alcool gras, et de préférence de 1 à 3% en poids.

5. Emulsion selon l'une des revendications 1 à 4, caractérisée en ce qu'elle comporte de 0,5 à 50% en poids par rapport au poids total de la composition
d'au moins un perfluoropolyéther, et de préférence de 5 à 20% en poids.

6. Emulsion selon l'une des revendications 1 à 5, caractérisée en ce qu'elle comporte de 10 à 90% en poids de phase aqueuse gélifiée, par rapport au poids total de la composition.

7. Emulsion selon l'une des revendications 1 à 6, caractérisée en ce que les perfluoropolyéthers sont choisis parmi :
- les composés de formule (I) : 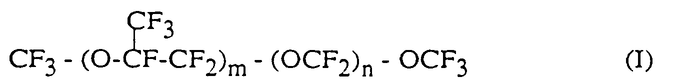 dans laquelle m/n = 5 à 40, le poids moléculaire moyen étant supérieur à 500 et de préférence compris entre 1.000 et 10.000,
- les composés de formule (II) :
CH₃-(O-CF₂-CF₂)ₚ-(OCF₂)_{q}-OCF₃ (II)
dans laquelle p/q est de 0,5 à 1,5, le poids moléculaire moyen étant supérieur à 500 et de préférence compris entre 1.000 et 10.000,
- les composés de formule (III) : 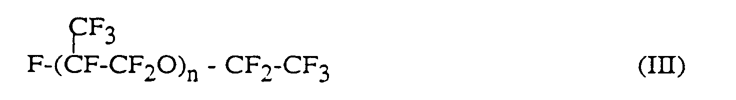 dans laquelle n est un nombre entier de 4 à 500,
- les composés de formule (IV) : 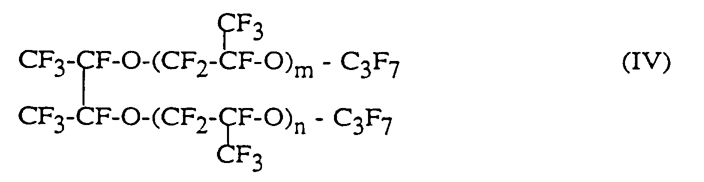 dans laquelle n et m sont des nombres entiers de 0 à 3, et
- les composés de formule (V) :
RCF₂-(O-CF₂CF₂)ₚ-(OCF₂)_{q}-OCF₂R (V)
dans laquelle :
p/q est de 0,5 à 1,5, et
R représente un reste -COOCH₃, -CH₂OH,
-CH₂O-CH₂-CHOHCH₂OH ou -CH₂-(OCH₂-CH₂)ₜ-OH où t est 1 ou 2, le poids moléculaire moyen étant supérieur à 500 et de préférence compris entre 1.000 et 10.000.

8. Emulsion selon l'une des revendications 1 à 7, caractérisée en ce qu'elle comporte à titre de co-émulsionnant hydrocarboné, un composé choisi parmi les éthers en C₄-C₂₀ polyoxyéthylénés et/ou polyoxypropylénés à chaîne hydrocarbonée et les copolymères oxyde d'éthylène-oxyde de propylène, et de préférence parmi les composés de formule : 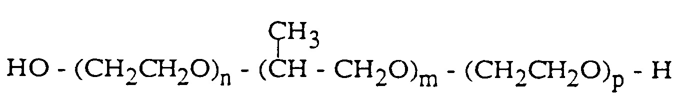 où m, n et p ont des valeurs de 2 à 100.

9. Emulsion selon l'une des revendications 1 à 8, caractérisée en ce que les tensio-actifs fluorés sont choisis parmi
- les composés de formule :
R_{f}-(CH₂)ₘ-S-Gₙ-H (VI)
dans laquelle :
. R_{f} désigne un radical alkyle perfluoré, linéaire ou ramifié, en C₆ à C₂₀ ou un mélange de radicaux alkyle perfluorés, linéaires ou ramifiés, en C₄ à C₂₀;
. m représente 0, 1 ou 2;
. n représente une valeur statistique ou entière comprise entre 1 et 10;
. G représente un motif choisi parmi : 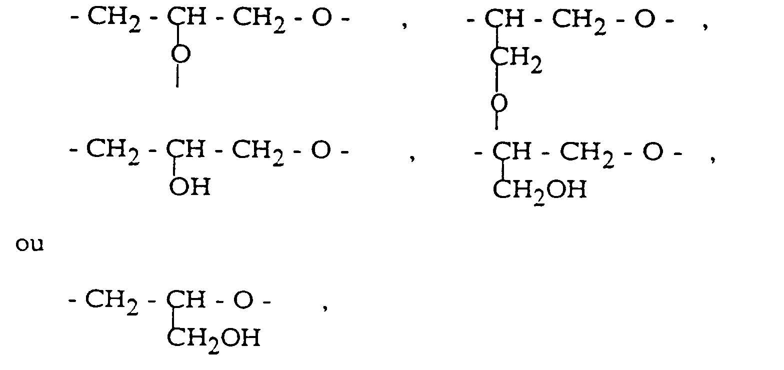
chacun des atomes d'oxygène pouvant être relié à un atome d'hydrogène ou à un autre motif G, et
- les composés de formule : 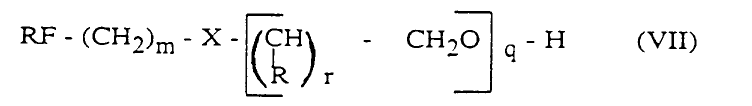 où RF est un groupement perfluoroalkyle CₙF₂ₙ₊₁ où n est compris entre 3 et 20;
. m = 0,1 ou 2;
. q = 1 à 10, de préférence 2;
. R est un groupement méthyle ou hydrogène;
. r = 1 lorsque R = CH₃;
. r = 1 ou 2 lorsque R = H; et
. X est un atome d'oxygène ou de soufre.

10. Emulsion selon l'une des revendications 1 à 9, caractérisée en ce que les gélifiants de la phase aqueuse gélifiée sont choisis parmi les celluloses modifiées comme l'hydroxyéthylcellulose, la méthylcellulose, l'hydroxypropylcellulose et la carboxyméthylcellulose, la gomme de caroube, la gomme de guar quaternisée ou non la gomme de xanthane, la gomme d'hydroxypropylguar, les acides polyacryliques réticulés, les polymères poly(meth)acrylates de glycéryl, la polyvinylpyrrolidone, l'alcool polyvinylique, les polymères réticulés d'acrylamide et d'acrylate d'ammonium ou d'acrylamide et d'acide 2-acrylamido-2-méthyl-propane sulfonique ou d'acrylamide et de methacryloyloxyéthyltriméthylammonium, et les homopolymères réticulés de chlorure de methacryloyloxyéthyltriméthylammonium.

11. Emulsion selon l'une des revendications 1 à 10, caractérisée en ce que les alcools gras sont choisis parmi les alcools à chaîne hydrocarbonée en C₁₂-C₂₂, de préférence l'alcool cétylique, le lauryl glycol et l'octyldodécanol.

12. Emulsion selon l'une des revendications 1 à 11, caractérisée en ce qu'elle comporte, dans la phase grasse, outre les perfluoropolyéthers et l'alcool gras, d'autres huiles telles que les huiles et cires hydrocarbonées; des esters d'acides gras; des triglycérides; des cires minérales, végétales, animales ou synthétiques, des silicones comportant éventuellement des groupes fonctionnels choisis parmi les fonctions alcool, amine ou thiol; des silicones fluorées; ou des huiles perfluorées.

13. Procédé de préparation d'une émulsion huile-dans-eau selon l'une des revendications 1 à 12, caractérisé en ce qu'on disperse le tensio-actif fluoré et le co-émulsionnant hydrocarboné dans la phase aqueuse gélifiée, on ajoute le gélifiant aqueux sous agitation vigoureuse, on chauffe le (ou les) perfluoropolyéther(s), l'alcool gras et les éventuels adjuvants de la phase grasse, et on incorpore la phase grasse dans la phase aqueuse sous agitation vigoureuse.

14. Utilisation des émulsions huile-dans-eau selon l'une des revendications 1 à 12, pour la préparation de compositions cosmétiques ou dermatologiques.

15. Composition cosmétique ou dermatologique comportant au moins une émulsion selon l'une des revendications 1 à 12.

16. Composition selon la revendication 15, caractérisée en ce qu'elle est sous forme de lait, de crème blanche, de crème de soin ou de crème anti-solaire, de crème teintée, de fond de teint ou de mascara.

## Claims

1. Oil-in-water emulsion, characterized in that it contains at least:
- one perfluoropolyether,
- one fluoro surfactant, with the exception of any cationic fluoro surfactant,
- one co-emulsifying agent,
- one fatty alcohol,
- a gelled aqueous phase.

2. Emulsion according to Claim 1, characterized in that it contains from 0.5 to 10% by weight of at least one fluoro surfactant relative to the total weight of the composition, and preferably from 1 to 3% by weight.

3. Emulsion according to Claim 1 or 2, characterized in that it contains from 0.1 to 5% by weight relative to the total weight of the composition of at least one hydrocarbon co-emulsifying agent, and preferably from 0.1 to 0.3% by weight.

4. Emulsion according to one of Claims 1 to 3, characterized in that it contains from 0.5 to 10% by weight relative to the total weight of the composition of at least one fatty alcohol, and preferably from 1 to 3% by weight.

5. Emulsion according to one of Claims 1 to 4, characterized in that it contains from 0.5 to 50% by weight relative to the total weight of the composition of at least one perfluoropolyether, and preferably from 5 to 20% by weight.

6. Emulsion according to one of Claims 1 to 5, characterized in that it contains from 10 to 90% by weight of gelled aqueous phase relative to the total weight of the composition.

7. Emulsion according to one of Claims 1 to 6, characterized in that the perfluoropolyethers are chosen from:
- the compounds of formula (I): 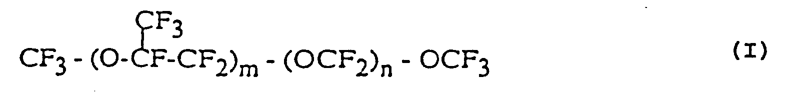 in which m/n = 5 to 40, the average molecular weight being higher than 500 and preferably between 1000 and 10,000,
- the compounds of formula (II):
CH₃-(O-CF₂-CF₂)ₚ-(OCF₂)_{q}-OCF₃ (II)
in which p/q is from 0.5 to 1.5, the average molecular weight being higher than 500 and preferably between 1000 and 10,000,
- the compounds of formula (III): 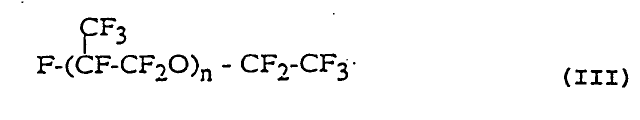 in which n is an integer from 4 to 500,
- the compounds of formula (IV): 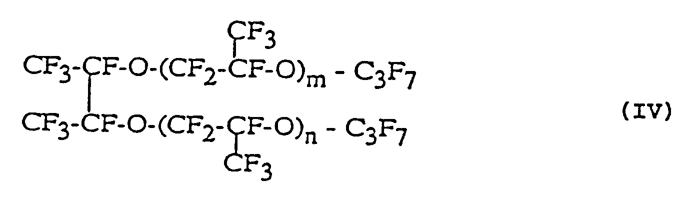 in which n and m are integers from 0 to 3, and
- the compounds of formula (V):
RCF₂-(O-CF₂CF₂)ₚ-(OCF₂)_{q}-OCF₂R (V)
in which:
p/q is from 0.5 to 1.5, and
R represents a residue -COOCH₃, -CH₂OH, -CH₂O-CH₂-CHOHCH₂OH or -CH₂-(OCH₂-CH₂)ₜ-OH where t is 1 or 2, the average molecular weight being higher than 500 and preferably between 1000 and 10,000.

8. Emulsion according to one of Claims 1 to 7, characterized in that it contains, as hydrocarbon co-emulsifying agent, a compound chosen from C₄-C₂₀ polyoxyethylenated and/or polyoxypropylenated ethers containing a hydrocarbon chain and ethylene oxide-propylene oxide copolymers, and preferably from the compounds of formula: 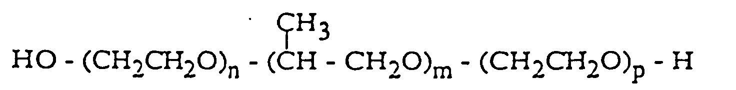 where m, n and p have values of from 2 to 100.

9. Emulsion according to one of Claims 1 to 8, characterized in that the fluoro surfactants are chosen from
- the compounds of formula:
R_{f}-(CH₂)ₘ-S-Gₙ-H (VI)
in which:
• R_{f} denotes a linear or branched C₆ to C₂₀ perfluoroalkyl radical or a mixture of linear or branched C₄ to C₂₀ perfluoroalkyl radicals;
• m represents 0, 1 or 2;
• n represents a statistical value or an integer between 1 and 10;
• G represents a unit chosen from: 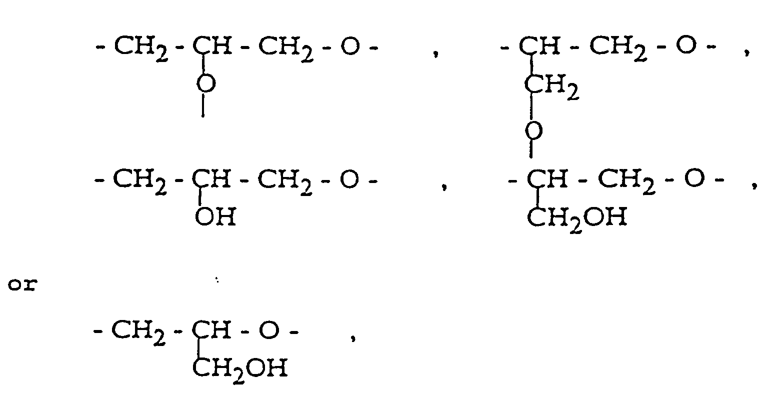
it being possible for each of the oxygen atoms to be linked to a hydrogen atom or to another unit G, and
- the compounds of formula: 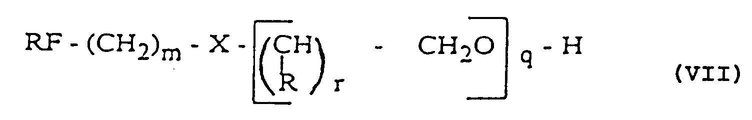 where
• RF is a perfluoroalkyl group CₙF₂ₙ₊₁ where n is between 3 and 20;
• m = 0, 1 or 2;
• q = 1 to 10, preferably 2;
• R is a methyl group or hydrogen;
• r = 1 when R = CH₃;
• r = 1 or 2 when R = H; and
• X is an oxygen or sulfur atom.

10. Emulsion according to one of Claims 1 to 9, characterized in that the gelling agents of the gelled aqueous phase are chosen from modified celluloses such as hydroxyethylcellulose, methylcellulose, hydroxypropylcellulose and carboxymethylcellulose, carob gum, quaternized or non-quaternized guar gum, xanthan gum, hydroxypropyl guar gum, crosslinked polyacrylic acids, polyglyceryl (meth)acrylate polymers, polyvinylpyrrolidone, polyvinyl alcohol, crosslinked polymers of acrylamide and ammonium acrylate or of acrylamide and 2-acrylamido-2-methylpropanesulfonic acid or of acrylamide and methacryloyloxyethyltrimethylammonium [lacuna], and crosslinked homopolymers of methacryloyloxyethyltrimethylammonium chloride.

11. Emulsion according to one of Claims 1 to 10, characterized in that the fatty alcohols are chosen from alcohols with a C₁₂-C₂₂ hydrocarbon chain, preferably cetyl alcohol, lauryl glycol and octyldodecanol.

12. Emulsion according to one of Claims 1 to 11, characterized in that it contains, in the fatty phase, besides the perfluoropolyethers and the fatty alcohol, other oils such as hydrocarbon oils and waxes; fatty acid esters; triglycerides; inorganic, plant, animal or synthetic waxes, silicones optionally containing functional groups chosen from alcohol, amine or thiol functions; fluorosilicones; or perfluoro oils.

13. Process for the preparation of an oil-in-water emulsion according to one of Claims 1 to 12, characterized in that the fluoro surfactant and the hydrocarbon co-emulsifying agent are dispersed in the gelled aqueous phase, the aqueous gelling agent is added with vigorous stirring, the perfluoropolyether(s), the fatty alcohol and the optional adjuvants of the fatty phase are heated, and the fatty phase is incorporated into the aqueous phase with vigorous stirring.

14. Use of the oil-in-water emulsions according to one of Claims 1 to 12 for the preparation of cosmetic or dermatological compositions.

15. Cosmetic or dermatological composition containing at least one emulsion according to one of Claims 1 to 12.

16. Composition according to Claim 15, characterized in that it is in the form of a milk, a white cream, a care cream or an antisun cream, a tinted cream, a foundation or a mascara.

## Patentansprüche

1. Öl-in-Wasser-Emulsion,
dadurch **gekennzeichnet**, daß
sie mindestens enthält:
- einen Perfluorpolyether,
- ein fluorhaltiges oberflächenaktives Mittel, ausgenommen jedes kationische fluorhaltige oberflächenaktive Mittel,
- einen Coemulgator,
- einen Fettalkohol,
- eine wässrige gelierte Phase.

2. Emulsion gemäß Anspruch 1,
dadurch **gekennzeichnet**, daß
sie 0,5 bis 10, vorzugsweise 1 bis 3, Gew.% mindestens eines fluorhaltigen oberflächenaktiven Mittels enthält, bezogen auf das Gesamtgewicht der Zusammenseztung.

3. Emulsion gemäß Anspruch 1 oder 2,
dadurch **gekennzeichnet**, daß
sie 0,1 bis 5, vorzugsweise 0,1 bis 0,3 , Gew.%, bezogen auf das Gesamtgewicht der Zusammensetzung, mindestens eines Kohlenwasserstoff-Coemulgators enthält.

4. Emulsion gemäß einem der Ansprüche 1 bis 3,
dadurch **gekennzeichnet**, daß
sie 0,5 bis 10, vorzugsweise 1 bis 3, Gew.%, bezogen auf das Gesamtgewicht der Zusammensetzung, mindestens eines Fettalkohols entählt.

5. Emulsion gemäß einem der Ansprüche 1 bis 4,
dadurch **gekennzeichnet**, daß
sie 0,5 bis 50, vorzugsweise 5 bis 20, Gew.%, bezogen auf das Gesamtgewicht der Zusammensetzung, mindestens eines Perfluorpolyethers enthält.

6. Emulsion gemäß einem der Ansprüche 1 bis 5,
dadurch **gekennzeichnet**, daß
sie 10 bis 90 Gew.% wässrige gelierte Phase umfaßt, bezogen auf das Gesamtgewicht der Zusammensetzung.

7. Emulsion gemäß einem der Ansprüche 1 bis 6,
dadurch **gekennzeichnet**, daß
die Perfluorpolyether ausgewählt sind aus:
- Verbindungen der Formel (I): 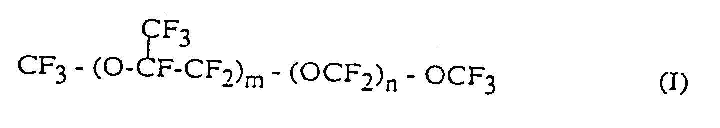 worin m/n = 5 bis 40, wobei das mittlere Molekulargewicht mehr als 500 und vorzugsweise 1000 bis 10000 beträgt,
- Verbindungen der Formel (II):
CH₃-(O-CF₂-CF₂)ₚ-(OCF₂)_{q}-OCF₃ (II)
worin p/q 0,5 bis 1,5 ist, wobei das mittlere
Molekulargewicht mehr als 500 und vorzugsweise 1000 bis 10000 beträgt,
- Verbindungen der Formel (III): 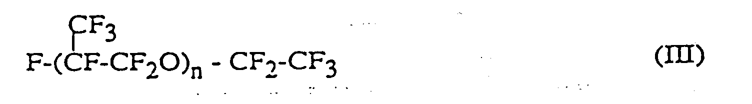 worin n eine ganze Zahl von 4 bis 500 ist,
- Verbindungen der Formel (IV): 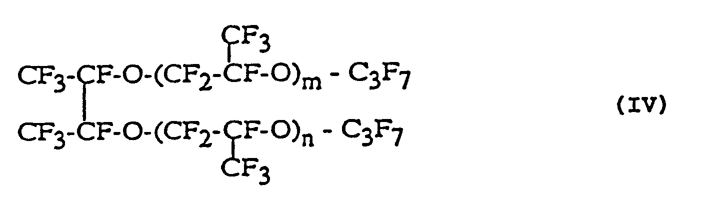 worin n und m ganze Zahlen von 0 bis 3 sind, und aus
- Verbindungen der Formel (V):
RCF₂-(O-CF₂CF₂)ₚ-(OCF₂)_{q}-OCF₂R (V)
worin gilt:
p/q ist 0,5 bis 1,5, und
R stellt einen Rest -COOCH₃, -CH₂OH, -CH₂O-CH₂-CHOHCH₂OH oder -CH₂-(OCH₂-CH₂)ₜ-OH dar, worin t 1 oder 2 ist,
und wobei das mittlere Molekulargewicht mehr als 500 und vorzugsweise 1000 bis 10000 beträgt.

8. Emulsion gemäß einem der Ansprüche 1 bis 7,
dadurch **gekennzeichnet**, daß
sie als Kohlenwasserstoff-Coemulgator eine Verbindung enthält, die aus polyexethylierten und/oder polyoxpropylierten C₄₋₂₀-Ethern mit Kohlenwasserstoffkette und aus Ethylenoxid-Propylenoxid-Copolymeren und vorzugsweise aus den Verbindungen der Formel ausgewählt ist: 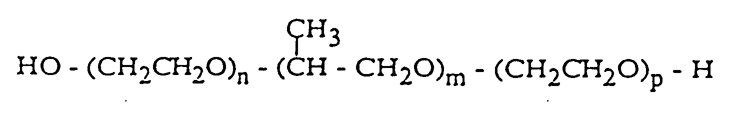 worin m, n und p Werte von 2 bis 100 aufweisen.

9. Emulsion gemäß einem der Ansprüche 1 bis 8,
dadurch **gekennzeichnet**, daß
die fluorhaltigen oberflächenaktiven Mittel ausgewählt sind aus:
- Verbindungen der Formel:
R_{f}-(CH₂)ₘ-S-Gₙ-H (VI)
worin gilt:
- R_{f} bedeutet einen linearen oder verzweigten C₆₋₂₀-Perfluoralkylrest oder eine Mischung aus linearen oder verzweigten C₄₋₂₀-Perfluoralkylresten;
- m stellt 0, 1 oder 2 dar;
- n stellt einen statistischen oder ganzzahligen Wert von 1 bis 10 dar;
- G stellt eine Einheit dar, ausgewählt aus: 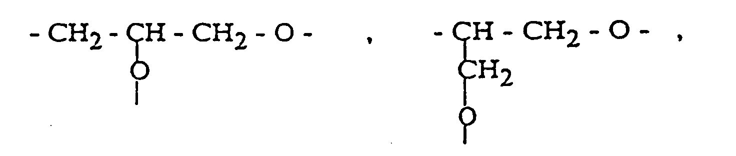 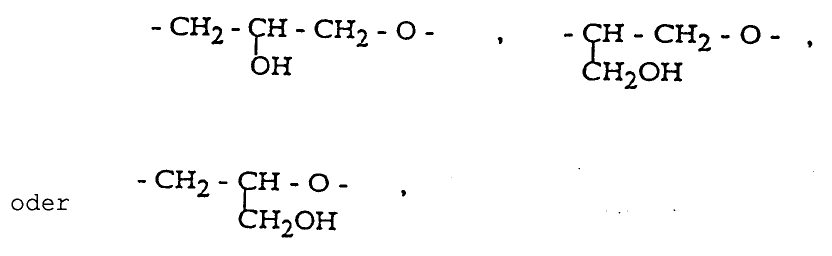 in denen jedes der Sauerstoffatome an ein Wasserstoffatom oder an eine weitere Einheit G gebunden sein kann, und aus
- Verbindungen der Formel: 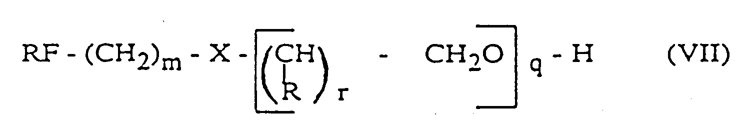 worin gilt:
- RF ist eine CₙF₂ₙ₊₁-Perfluoralkylgruppe, worin n 3 bis 20 beträgt;
- m = 0, 1 oder 2, vorzugsweise 2;
- q = 1 bis 10, vorzugsweise 2;
- R ist eine Methylgruppe oder Wasserstoff;
- r = 1, wenn R = CH₃, und r ist 1 oder 2, wenn R = H;
- X ist ein Sauerstoff- oder Schwefelatom.

10. Emulsion gemäß einem der Ansprüche 1 bis 9,
dadurch **gekennzeichnet**, daß
die Geliermittel der wässrigen gelierten Phase aus modifizierten Cellulosen wie Hydroxyethylcellulose, Methylcellulose, Hydroxypropylcellulose und Carboxymethylcellulose, aus Johannisbrot-Gummi, Guar-Gummi, quaterniert oder nicht, Xanthan-Gummi, Hydroxypropylguar-Gummi, aus vernetzten Polyacrylsäuren, aus Glycerylpoly(meth)acrylat-Polymeren, Polyvinylpyrrolidon, Polyvinylalkohol, vernetzten Polymeren aus Acrylamid und Ammoniumacrylat oder Acrylamid und 2-Acryamido-2-methylpropansulfonsäure oder Acrylamid und Methacryloyloxyethyltrimethylammoniumchlorid und aus vernetzten Homopolymeren aus Methacryloyloxyethyltrimethylammoniumchlorid ausgewählt sind.

11. Emulsion gemäß einem der Ansprüche 1 bis 10,
dadurch **gekennzeichnet,** daß
die Fettalkohole aus Alkoholen mit C₁₂₋₂₂-Kohlenwasserstoffkette, vorzugsweise aus Cetylalkohol, und aus Laurylglycol und Octyldodecanol ausgewählt sind.

12. Emulsion gemäß einem der Ansprüche 1 bis 11,
dadurch **gekennzeichnet**, daß
sie, in der Fettphase, ausser den Perfluorpolyethern und dem Fettalkohol weitere Öle wie Kohlenwasserstofföle und -wachse, Fettsäureester, Triglyceride, mineralische, pflanzliche, tierische oder synthetische Wachse, Silicone, gegebenenfalls mit funktionellen Gruppen aus Alkohol-, Amin- oder Thiol-Funktionen, fluorhaltige Silicone oder perfluorierte Öle enthält.

13. Verfahren zur Herstellung einer Öl-in-Wasser-Emulsion gemäß einem der Ansprüche 1 bis 12,
dadurch **gekennzeichnet**, daß
man das fluorhaltige oberflächenaktive Mittel und den Kohlenwasserstoff-Coemulgabor in der wässrigen gelierten Phase dispergiert, das wässrige Geliermittel unter kräftigem Rühren zufügt, den oder die Perfluorpolyether, den Fettalkohol und die gegebenenfalls vorhandenen Hilfsstoffe der Fettphase erwärmt und die Fettphase in die wässrige Phase unter kräftigem Rühren einbringt.

14. Verwendung von Öl-in-Wasser-Emulsionen gemäß einem der Ansprüche 1 bis 12 zur Herstellung kosmetischer oder dermatologischer Zusammensetzungen.

15. Kosmetische oder dermatologische Zusammensetzung, enthaltend mindestens eine Emulsion gemäß einem der Ansprüche 1 bis 12.

16. Zusammensetzung gemäß Anspruch 15,
dadurch **gekennzeichnet**, daß
sie in Form einer Milch, weißen Creme, Pflege- oder Sonnenschutzmittel-Creme, gefärbten Creme, Teint-Grundlage oder Wimperntusche vorliegt.
